Europäisches Patentamt

(19) European Patent Office

Office européen des brevets

(11) Publication number: **0 220 849 B1**

# EUROPEAN PATENT SPECIFICATION

(45) Date of publication of patent specification:
27.02.91 Bulletin 91/09

(51) Int. Cl.⁵: **A61K 33/08,** // (A61K33/08, 33:00, 31:19)

(21) Application number: 86307619.6

(22) Date of filing: 02.10.86

(54) Pharmaceutical compositions having cytoprotective properties.

The file contains technical information submitted after the application was filed and not included in this specification

(30) Priority: 08.10.85 US 785417
20.02.86 US 831756

(43) Date of publication of application:
06.05.87 Bulletin 87/19

(45) Publication of the grant of the patent:
27.02.91 Bulletin 91/09

(84) Designated Contracting States:
AT BE CH DE FR GB IT LI LU NL SE

(56) References cited:
FR-A- 2 103 569
CHEMICAL ABSTRACTS, vol. 80, no. 26, 1st July 1974, pages 193-194, no. 149115a, Columbus, Ohio, US; & JP-A-74 13 319
CHEMICAL ABSTRACTS, vol. 98, no. 17, 25th April 1983, page 47, no. 137460t, Columbus, Ohio, US; I. SZELENYI: "Cytoprotection with antacids"

(73) Proprietor: AMERICAN HOME PRODUCTS CORPORATION
685, Third Avenue
New York, New York 10017 (US)

(72) Inventor: Borella, Luis Enrique
5 Jacob Drive
Lawrenceville New Jersey (US)
Inventor: Dijoseph, John Francis
571 Linden Avenue
Woodbridge New Jersey (US)
Inventor: Mir, Ghulam Nabi
P.O. Box 392
Buckingham Pennsylvania 18912 (US)
Inventor: Reuter, Gerald Louis
23 Crescent Drive
Plattsburgh New York 12901 (US)

(74) Representative: Porter, Graham Ronald et al
C/O John Wyeth & Brother Limited
Huntercombe Lane South Taplow
Maidenhead Berkshire, SL6 0PH (GB)

Jouve, 18, rue Saint-Denis, 75001 PARIS

## Description

This invention relates to exogenously acidified antacid compositions having gastric cytoprotective properties. More particularly this invention relates to exogenously acidified aluminium base containing antacid compositions wherein an antacid suspension has been acidified to the point at which the aluminium ion component is solubilized, and then formulated into liquid dosage forms or for example, spray dried and formulated into solid dosage forms.

Gastric cytoprotection not involving the inhibition of gastric acid secretion, is a known phenomenon. For example, prostaglandin F2 does not inhibit gastric acid secretion, but the compound does induce gastric cytoprotection. Other prostaglandins induce gastric cytoprotection at much smaller dose levels than those required for the inhibition of gastric acid secretion. See for example, Shriver, U.S.-A-. 4,370,348.

Although the mechanism of cytoprotection by antacids is not clearly defined yet, there is a suggestion that it may be partially mediated through the release of gastric mucosal prostglandins, (Hollander et al. Gastroenterology 86 : 1114, 1984 and Tarnowski et al, Gastroenterology 86 : 1276, 1984). Szelenyi et al, (Gastroenterology 88 : 1604, 1985) has suggested non-prostaglandin mediated mechanisms for cytoprotection.

Activity in the ethanol induced ulcer model is an indication of cytoprotection, regardless of the antisecretory characteristics of the drug. Antisecretory agents, such as the $H_2$ receptor antagonist cimetidine and the anticholinergic agent propantheline bromide do not protect this model. See Robert et al, Scand. J. Gastroenterol. 19 (Suppl. 101) : 69, 72, 1984.

The cytoprotective activity of antacids is a recent observation (Hagel et al, Hepato-gastroenterol. 29 : 271-274, 1982. Szelenyi et al, Eur. J. Pharmacol. 88 : 403-406, 1983. Hollander et al, Gastroenterology 86: 1114, 1984. For example, it has been shown by Szelenyi et al, Gastroenterology 88 : 5 Part 2, 1604 (1985) and Tarnowski et al, Gastroenterology 86 : 5, Part 2, 1276 (1985) that Al(OH), MAALOX and MYLANTA have cytoprotective properties. MAALOX and MYLANTA are registered trade markes.

We have demonstrated that magaldrate and other commercially available aluminium base containing antacids inhibit ethanol induced ulcers in rats. The activity of acidified magaldrate in this test suggests, therefore, that it possesses cytoprotective properties as an addition to its acid neutralizing effects.

Antacids have long been thought to exert their antiulcer effects primarily by one of the following mechanisms : 1) acid neutralization, 2) inactivation of pepsin (Piper et al, Am. J. Dig, Dis 6 (2) : 134-141, 1961) and 3) binding to bile salts (Beneyto et al, Arzneim.-Forsch 34 (11) : 1350-1354, 1984). The coating of the ulcer crater by antacids has also been considered, but it is not a viable mechanism (Piper, Clinics in Gastro. 2 (2) : 361-377, 1973).

In order to substantiate the cytoprotective activity of magaldrate, as distinguished from the other mechanisms of antiulcer activity, 6 N HCl was added to magaldrate to negate its acid neutralization capacity. The pH was changed from approximately 9.0, for a commercial magaldrate suspension to pH 2.5, for acidified magaldrate source. At pH 2.5. acidified magaldrate was significantly more potent in preventing ethanol-induced ulcers in the rat than the commercial magaldrate formula. Therefore, acidified magaldrate fulfills Robert's criteria for a cytoprotective agent, i.e., antiulcer effects at doses which are not antisecretory. Also, the acidified magaldrate was used at low pH (<3.0) in a solubilized form in which antacids are reported not able to inactivate pepsin (Wenger et al, J. Clin. Pharmacol. 12 : 136-141, 1972.) In addition, bile salts are not reported to be involved in ethanol induced ulceration and bile is not visibly present in ethanol treated rat stomachs. Since acid neutralization, pepsin inactivation, and bile binding are not involved in the antiulcer activity of acidified magaldrate in the ethanol model, the contribution of the three viable antiulcer mechanisms to the antiulcer effects of acidified magaldrate has been eliminated and the antiulcer effect of acidified magaldrate can therefore be attributed to its cytoprotective effects.

The above identified article by Wenger et al, J. Clin. Pharmacol. 12 : 136-141, 1972 entitled "Pepsin Adsorption By Commercial Antacid Mixtures. In Vitro Studies" described the adsorption of pepsin by various commercial antacid mixtures independently of their effect on pH. In the experiments, antacid dilutions were prepared by adding 10 grams of certain commercial antacid suspensions to 100 milliliters of distilled water. Using hydrochloric acid and distilled water, the concentration was further decreased to 5 grams per 100 milliliters. For each mixture four flasks were prepared and the final pH of each was brought to 1.5, 3.0, 5.0 and 6.0. The antacids included Gelusil-M, Delcid, Maalox, Riopan and Amphojel (registered trade marks). The article refers to other authors who have similarly acidified antacid solutions.

According to the invention there is provided a cytoprotective pharmaceutical composition characterised in that the composition comprises an exogenously acidified aluminium base containing composition acidified to a pH at which the aluminium base component is solubilized and having a concentration of 50 to 98 grams of precursor aluminium base containing composition prior to acidification per 100 milliliters of acidified composition. The aluminium base containing compositions may comprise for example, commercial liquid antacid suspensions containing an aluminium base such as aluminium hydroxide, antacid powders or solids containing an aluminium base such as aluminium hydroxide, or aluminium hydroxide gel, such compositions may include magnesium hydroxide. The end point for the solubilization is ordinarily in the range of pH 2.25 to 3.25

In another embodiment of this invention, the exogenously acidified compositions containing solubilized aluminium base are further formulated into liquid dosage forms such as syrups, or for example, the solutions are spray dried and formulated into solid dosage forms such as powders for encapsulation or compression into tablets.

Aluminium hydroxide is aluminium hydrate, aluminium trihydrate or hydrated alumina of formula $Al(OH)_3$. The aluminium hydroxide is described only as a gastric antacid (see the Merck Index, 8th Edition p 44).

Acidified aluminium hydroxide, according to the present invention, is produced by treating an aqueous gel or suspension of aluminium hydroxide with an acid such as aqueous hydrochloric acid until the suspension becomes solubilized.

Magaldrate is a magnesium aluminate hydrate, described in Hallmann et al, U.S.-A-2,923,660. Extra strength magaldrate and rehydratable magaldrate are disclosed and claimed in European Patent Application 0178895.

Magaldrate is a chemical union of aluminium and magnesium hydroxide, corresponding approximately to the formula $Al_5Mg_{10}(OH)_{31}(SO_4)_2xH_2O$ according to the official monograph USP XX, third supplement USP-NF and has a molecular weight of about 1097. Magaldrate, also sometimes referred to in said monograph as aluminium magnesium hydroxide sulfate, contains not less than 29.0 per cent and not more than 40.0 percent of magnesium oxide ($M_gO$) and the equivalent of not less than 18.0 percent and not more than 26.0 percent of aluminium oxide ($Al_2O_3$).

The preparation of magaldrate is described in U.S.-A-2,923,660. A commercially suitable procedure is described in said patent, for example, beginning in column 2, line 40. Aluminium sulfate is employed as at column 2, line 58 in order to obtain a magaldrate "all sulfate" material and, to maintain a low sodium content for the final product, the use of potassium oxide (or hydroxide) is preferred over the disclosed sodium oxide. Typically the magaldrate is precipitated to provide a 6% weight/volume mixture (fluid when fresh) and diluted to 3% for washing prior to concentration and formulation into a suspension providing a so called single strength acid neutralization capacity (ANC) of 13.5 to 15 meq per 5 milliliters of suspension which is equivalent to a magaldrate weight/weight concentration in the range of about 12 to 13 percent solids. At this concentration, unformulated magaldrate is a paste-like gel. Formulated magaldrate is sold under the RIOPAN trademark.

In addition to aluminium hydroxide gel and magaldrate gel described above, suitable starting materials also include other aluminium hydroxide-magnesium hydroxide gels prior to formulation into commercial antacid compositions and spray dried powders formed from such gels.

Surprisingly, antacid compositions based on aluminium phosphate, when acidified, do not exhibit cytoprotective activity. Also, aluminium chloride does not exhibit such activity.

Suitable commercial liquid antacids for preparing the acidified compositions of this invention include for example, AMPHOJEL registered trade marks and ALUDROX marketed by Wyeth Laboratories Inc. of Philadelphia, Pennsylvania, RIOPAN, RIOPAN PLUS and RIOPAN Extra Strength marketed by Ayerst Laboratories Inc. New York, N.Y., MAALOX, MAALOX PLUS and MAALOX TC marketed by William H. Rorer Inc. Fort Washington, Penn., MYLANTA and MYLANTA II marketed by Stuart Pharmeceuticals, Wilmington, Delaware, and DI-GEL marketed by Plough Inc., Memphis Tenn.

MAALOX Suspension (manufactured by Rorer, Physicians' Desk Reference for Nonprescription Drugs 6th ed. 1985) is a combination of magnesium and aluminium hydroxides containing 225 mg aluminium hydroxide equivalent to dried gel USP, and 200 mg magnesium hydroxide per 5 ml of suspension. Each 5 ml of MAALOX Suspension neutralizes 13.3 mEq of acid. MYLANTA (manufactured by Stuart, Physicians' Desk Reference for Nonprescription Drugs 6th ed. 1985) is a liquid containing 200 mg aluminium hydroxide, 200 mg magnesium hydroxide and 20 mg simethicone per 5 ml of liquid. Each 5 ml of MYLANTA liquid neutralizes 12.7 mEq. of acid.

The acidification of the antacids should be accomplished with care so that the end point does not drop much below the pH at which the suspension becomes a solution or at which the aluminium hydroxide is solubilized. This is to minimize formation of other aluminium species. Thus the gel preferably is diluted with water or the solid rehydrated with water prior to acidification.

Suitable acids for acidification include the mineral acids hydrochloric, nitric and sulfuric acids, phosphoric acid and the organic acids such as acetic acid, lactic acid, citric acid and propionic, glycolic, lactic, adipic and maleic acids. If the organic acids do not give a clear to translucent solution at a pH of about 2.25 to about 3.25, then the addition of a small amount of hydrochloric acid usually will provide a near clear or translucent solution. Sulfuric acid in high concentration causes an exothermic reaction sufficient to char the antacid suspensions so must be used with care.

This invention is directed to the use of acidified antacids as cytoprotective agents. Cytoprotection mechanisms are not well defined. However, it is clear that lower dosages of cytoprotective agents are required for effectiveness than are required to inhibit gastric acid secretions. Because of its cytoprotective nature, exogenously acidified antacids may be used to treat or prevent disease states such as gastric and duodenal ulcers, regional ileitis, Crohn's disease, erosive gastritis, erosive esophagitis, inflammatory bowel disease and ethanol-induced hemorrhagic erosions.

The acidified antacid compositions may be administered alone or concomitantly or in combination with another active drug including an $H_2$ blocker such as cimetidine and ranitidine, an antispasmotic such as a belladonna alkaloid, or another medicinal product which may be irritating to the gastrointestinal tract such as aspirin®, ibuprofen, acetaminophen, and azulene.

Any suitable dosage form may be employed for providing a mammal, especially a human with the effective dosage of acidified antacid. For example, suitable dosage forms include tablets, dispersions, suspensions, solutions, suppositories, or capsules for oral, parenteral or rectal administration.

A convenient dosage form is to administer directly the acidified antacid solution. However, in practice the acidified antacid solution may be spray dried. The dry powder can be combined as the active ingredient in intimate admixture with a pharmaceutical carrier according to conventional pharmaceutical compounding techniques. The carrier may take a wide variety of forms depending on the form of preparation desired for administration.

In preparing the compositions for oral dosage form, any of the usual pharmaceutical media may be employed, such as, for example, water, glycols, oils, flavoring agents, preservatives, or coloring agents in the case of oral liquid preparations, such as for example, suspensions, emulsions and solutions ; or carriers such as starches, sugars, diluents, granulating agents, lubricants, binders, or disintegrating agents in the case of oral solid preparations such as, for example, powders, capsules and tablets. Because of their ease of administration, tablets and hard or soft gelatin capsules represent the most advantageous oral dosage unit form, in which case solid pharmaceutical carriers are obviously employed. If desired, tablets may be sugar coated or otherwise coated by standard techniques.

In addition to acidified antacid the pharmaceutical composition may also contain other active ingredients, such as nonsteroidal anti-inflammatory agents e.g., etodolac, indomethacin, ibuprofen, sulindac, fenbufen, or peripheral analgesic agents such as zomepirac, diflunisal.

EXAMPLE 1

A magaldrate suspension product according to Example 4 of EP-A-0178895 having an ANC of 30 meq/5 ml of magaldrate, 1080 mg/5 ml of suspension was prepared as follows. The formula given is for 1 liter (1.18 kg). This is the approximate formula for commercial RIOPAN Extra Strength suspension.

| Ingredient | Amount |
|---|---|
| Magaldrate Gel (sulfate, potassium base) | 2.93 l* |
| Aluminium Hydroxide Gel(12.5%$Al_2O_3$) | 47.8 g |
| Potassium Citrate | 19.6 g |
| Sorbitol Solution (70%) | 57.4 g |
| Glycerin | 47.8 g |
| Saccharin | 0.383 g |
| Xanthan Gum | 1.43 g |
| Peppermint, Natural and Artificial | 0.283 ml |
| Monochloramine Solution (sanitizer) | q.s. sufficient quantity to give 100 p.p.m. |
| Water, purified, Chlorinated q.s. | 1.18 Kg |

*Theoretical input per liter is 216g Magaldrate at 100%

1. In a suitable tank equipped with a mixer, the sorbitol solution, 15.7 g of the water and the citrate were combined and then mixed until a clear solution was obtained. The aluminium hydroxide gel was added and mixed until uniform and mixing continued until use.

2. Immediately before concentration of the magaldrate gel 126 g of the Step 1 mixture was added to a

4

jacketed tank equipped with a stirrer. With continuous stirring, the magaldrate gel was concentrated on a rotary filter to a concentration of not less than 24% w/w into the mixture and cooling to 25°C was begun. After all the concentrated antacid had been added the remainder of the Step 1 mixture was added with continuous stirring to achieve uniformity.

3. The Step 2 mixture was processed through a homogenizer into a jacketed tank with continuous stirring and continuous cooling to 25°C.

4. The remaining ingredients, except the water and monochloramine, were mixed in a separate container until uniform, after which, they were added to the magaldrate mixture and mixed until the xanthan gum was hydrated. Water was then added (with mixing) to bring the batch up to about 1.18 kg.

The acidified extra strength magaldrate of this invention was prepared by adding 6 N HCl to an endpoint of pH 2.5 as determined by a Corning®, Model 7, pH meter. Equilibration was achieved over 12 hours. At this endpoint, the antacid suspension became solubilized. This indicates a dissolution of the active antacid species. The dose volume of antacid was adjusted for the volume of 6N HCl added to the suspension.

## EXAMPLE 2

Acidified MAALOX of the present invention was prepared by adding to a sample of commercial MAALOX 6 N HCl to an endpoint of pH 3.0 as determined by a Corning®, Model 7, pH meter. Equilibration was achieved over 12 hours. At this endpoint, the antacid suspension became solubilized indicating a dissolution of the active antacid species.

## EXAMPLE 3

Acidified MYLANTA, of the present invention, was produced by treating a sample of commercial MYLANTA liquid with 6 N hydrochloric acid until a pH of 3.0. At this pH the antacid suspension became solubilized, again indicating a dissolution of the active antacid species.

## EXAMPLE 4

Aluminium hydroxide gel of commercial grade was admixed with and suspended in water to yield a suspension containing the equivalent of 133 mg $Al_2O_3$ per ml. The suspension was then acidified with 6N HCl until the aluminium hydroxide was solubilized at an end point of pH 3.0. A clear solution resulted.

## EXAMPLE 5

A sample of commercial BASALJEL (registered trade mark) marketed by Wyeth Laboratories, Inc., Philadephia, Penn., was acidified by adding 6N HCl to an endpoint of pH 2.5 as determined by a Corning Mode 7 pH meter. BASALJEL contains dried basic aluminium carbonate end the acidification was accompanied by copious gas evolution. The suspension prior to acidification contained the equivalent of 200 mg aluminium hydroxide per ml.

## EXAMPLE 6

A sample of commercial PHOSPHALJEL (registered trade mark) marketed by Wyeth Laboratories, Inc., of Philadelphia, Penn. was acidified by adding 6N HCl to an endpoint of pH 2.5. PHOSPHALJEL contains 46.6 mg aluminium phosphate per ml.

## EXAMPLE 7

The cytoprotective activity of a compound may be observed in both animals and man by noting the increased resistance of the gastrointestinal mucosa to the noxious effects of strong irritants, for example, the ulcerogenic effects of aspirin or indomathacin. In addition to lessening the effect of non-steroidal anti-inflammatory drugs on the gastrointestinal tract, animal studies show that cytoprotective compounds will prevent gastric lesions induced by oral administration of strong acids, strong bases, ethanol and, hypertonic saline solutions.

The ethano-induced lesion asssy is a standard test for cytoprotective activity end is described in an article by A. Robert at al entitled "Cytoprotection By Prostaglandins in Rats" published in Gastroenterology 77 : 433-443, 1979.

The ethanol-induced lesion assay was used to measure the cytoprotective ability of commercial antacids. Male Sprague-Dawley rats supplier, Charles River, Willmington, MA) weighing between 120-150 g were used. The rats were fasted for 24 hr prior to use (water ad libitum). Before dosing, the animals were placed two per cage end denied water.

Animals were orally, i.e. intragastrically pretreated, one hour before ethenol administration with either RIOPAN Extra Strength suspension, MAALOX suspension, $PGE_2$, or vehicle. One hour after ethanol administration, the rats were sacrificed by $CO_2$ asphyxiation. The stomachs were removed and kept moist with saline until the lesions were scored. Ulcers were scored with the investigator unaware of the treatment groups (single blind). Ulcers were graded according to the following scale :

| **GRADE** | **DESCRIPTION** |
|---|---|
| | (Approximate length of lesion) |
| 0 | no lesion |
| 1 | 2 mm or less |
| 2 | 4 mm |
| 3 | 6 mm |

Absolute ethenol was given orally, 1 ml/rat. RIOPAN Extra strength, 1080 mg magaldrate per 5 ml (lot 3RM4), and MAALOX, 200 mg $M_g OH)_2$+225 mg $Al(OH)_3$ per 5 ml (lot 64700), were administered orally as commercially available suspensions. The dose volume administered was based on the $ED_{50}$ (ml of antacid/kg, see Table 1).

The dose volume of acidified antacid was adjusted for the volume of 6 N HCl added to the suspension. $PGE_2$ was stored in 100% ethanol and diluted with normal saline before use.

Magaldrate of Example 1 end MAALOX of Example 2 were prepared as described above. The dose volume of nonbuffering antacid was adjusted for the volume of 6 N HCl added to the suspension during preparation. $PGE_2$ was stored in 100% ethanol end diluted with normal saline before use.

RESULTS

The mean ulcer score of each treatment group was compared to the control group and expressed as the percent inhibition of ulcer formation.

The cytoprotective $ED_{50}$ before and after acidification for the magaldrate of Example 1, referred to in this example as RIOPAN Extra Strength, and for MAALOX of Example 2 was calculated by standard regression analysis of the dose-response data. Confidence limits of 95% were calculated using the statistical differential method.

RIOPAN Extra Strength and the MAALOX of Example 2 respectively prior to acidification each produced a dose dependent reduction in ulcer score (Table 1). When calculated on the basis of weight of antacid administered, the $ED_{50}$ for magaldrate and MAALOW were not significantly different 453.6 mg/kg for magaldrate and 374 mg/kg for MAALOX, (Table 1).

At pH 2.5, RIOPAN Extra Strength was solubilized in accordance with this invention. It produced a dose dependent reduction in ulcer score ($ED_{50}$=58.3 mg/kg). Acidified magaldrate was significantly more potent than commercial magaldrate in inhibiting ulcer formation (Table 1).

MAALOX was solubilized at pH 3.0 in accordance with this invention. The solubilized, acidified MAALOX reduced the ulcer score dose dependently and was significantly more potent than commercial MAALOX (Table 1). There was no statistical difference in the antiulcer $ED_{50}$'s calculated for acidified magaldrate and acidified MAALOX (Table 1).

Effect of regular and acidified RIOPAN Extra Strength suspension and MAALOX suspension on ethanol induced gastric ulcers in the rat as discussed above is shown in Table 1 below :

## T A B L E  I

| Antacid | Dose: Per Os ml Antacid Suspension per Kg | MG ANTACID per Kg | % Inhibition | $ED_{50}$=mg/kg (95%C)* |
|---|---|---|---|---|
| RIOPAN Extra Strength (commercial) | 8 | 1728 | 76 | 453.6 |
| | 2 | 432 | 42 | (237.6-799.2) |
| | 0.5 | 108 | 30 | |
| RIOPAN Extra Strength (acidified) | 1 | 216 | 86 | 58.3 |
| | 0.5 | 108 | 77 | (38.9-86.4) |
| | 0.25 | 54 | 44 | |
| MAALOX (commercial) | 20 | 1700 | 85 | 374 |
| | 5 | 425 | 51 | (212.5-646.0) |
| | 1 | 85 | 18 | |
| MAALOX (acidified) | 1 | 85 | 76 | 43.4 |
| | 0.3 | 25.5 | 24 | (28.1-63.8) |
| | 0.1 | 8.5 | 3 | |

*Cytoprotective $ED_{50}$

It was found that RIOPAN Extra Strength and MAALOX dose dependently reduced ethanol-induced gastric ulcers in the rat ($ED_{50}$=453.6 mg/kg, p.o., and $ED_{50}$=374.0 mg/kg, p.o., respectively). The recommended human dose of RIOPAN Extra Strength (10 ml) corresponds to an oral dose of 43 mg/kg of magaldrate, and for MAALOX (20 ml), an oral dose of 34 mg/kg of antacid for a 50 kg person. RIOPAN Extra Strength and MAALOX were acidified in accordance with this invention by the addition of 6 N HCl to an endpoint of pH 2.5 for RIOPAN Extra Strength and pH 3.0 for MAALOX. Acidified RIOPAN Extra Strength and MAALOX dose dependently reduced ethanol-induced gastric ulcers ($ED_{50}$=58.3 mg/kg, p.o., and $ED_{50}$=43.4 mg/kg, p.o., respectively) Acidified RIOPAN Extra Strength and acidified MAALOX were about eight fold more potent than commercial RIOPAN Extra Strength and MAALOX in inhibiting ethanol-induced ulcers in rat. RIOPAN Extra Strength and MAALOX were cytoprotective in the ethanol-induced gastric cytotoxicity model in rat.

When tested at their $ED_{50}$ doses, both RIOPAN Extra Strength and MAALOX, commercial and acidified, significantly inhibited ethanol induced gastric ulcers when compared with vehicle (physiological saline) treated rats (Table 2).

# T A B L E  2

Effect of the oral ED$_{50}$ dose of commercial and acidified RIOPAN Extra Strength and MAALOX on ethanol induced gastric ulcers in rat.

| ANTACID | DOSE, MG/KG P.O. | % INHIBITION |
|---|---|---|
| RIOPAN Extra Strength (commercial) | 453.6 | 61 * |
| RIOPAN Extra Strength (acidified) | 58.3 | 50 * |
| MAALOX (commercial) | 374.0 | 60 * |
| MAALOX (acidified) | 43.4 | 56 * |

* $p < .01$, significantly different from vehicle treated group, Dunnett's multiple comparison test.

Examples 4 through 6 are summarized with respect to antacid content in Table 3.

## T A B L E   3

| Antacid | Dose: Per Os ml Antacid Suspension per Kg | % Inhibition | ED$_{50}$=mg/kg (95%C)* |
|---|---|---|---|
| Aluminium | 0.1 | 6 | 66.5 |
| Hydroxide Gel | 0.5 | 65 | |
| (Example 4) | 2.0 | 74 | |
| BASALJEL | 0.1 | 30 | 120 |
| Extra Strength | 0.5 | 65 | |
| (Example 5) | 2.0 | 73 | |
| PHOSPHALJEL | 0.5 | 12 | 14 |
| (Example 6) | 2.0 | 25 | |
| | 8.0 | 65 | |

Cytoprotective ED$_{50}$

It can be seen from these data that the acidified aluminiumphosphate containing antacid is relatively weak in cytoprotective activity as compared with the products of Examples 4 and 5. This is probably because it contains less basic aluminium than the others.

PGE$_2$ inhibited ethanol induced ulcer formation (Table 4). PGE$_2$ was approximately 1000 times more potent than either acidified RIOPAN Extra Strength or acidified MAALOX.

## T A B L E   4

Effect of PGE$_2$ on ethanol induced gastric ulcers in rat.

| | DOSE, g/kg P.O. | % INHIBITION | ED$_{50}$ g/kg (95% CI) |
|---|---|---|---|
| PGE$_2$ | 75 | 80 | 34.1 |
| | 50 | 63 | (27.3–42.3) |
| | 25 | 39 | |

As indicated above, inducement of gastric cytoprotection by acidified antacids is unrelated to the inhibition of gastric acid secretion. Although the mechanism of gastric cytoprotection is unknown, it appears that cytoprotective acidified antacids increase the resistance of gastric mucosal cells to the necrotizing effect of strong irritants. It is suggested, therefore, that nonbuffering antacids by a mechanism other than inhibition of gastric acid secretion, maintain the integrity of the gastric mucosa and will thus be beneficial in the treatment of those disease states wherein injury to the gastric mucosa is present.

Based on the data obtained in these assays, the effective oral dosage level to obtain cytoprotective effects from the exogenously acidified aluminium hydroxide containing compositions of this invention derived from commercial antacids is about one-eighth the oral dosage level of the active antacid component or ingredients of the respective commercial antacid. Such oral dosage levels of commercial antacids appear on their labels and generally range from 200 to 400 milligrams of aluminium hydroxide per dose or 400 to 600 milligrams of magaldrate per dose. Accordingly the dosage levels of the liquid and solid cytoprotective compositions of this invention can range from about one-eighth to the equivalent dose of the precursor antacid or aluminiumbase.

## EXAMPLE 8

In the following examples, acidified antacids similar to those of Examples 1 through 4 were spray dried. In these examples a Buchi® 190 Mini Spray Dryer was employed to dry the acidified antacids. This model spray dryer is manufactured by Buchi® Laboratoriums-Technik AG. Other spray dryers can be employed, however, such as those manufactured by Anhydro Company of Attleboro, Massachusetts and Niro Atomizer Inc., of Columbia, Maryland, so long as the spray dryer can process he relatively viscous admixture. The operating conditions for the Buchi® 190 Mini Spray Dryer are customarily an inlet temperature of 220°C, and an outlet temperature of 130°C. Since the acidified antacids are corrosive, special nozzles should be employed such as those fabricated from titanium.

In this example, (12 fl oz (US)) 355 ml (of commercial MAALOX TC suspension were acidified with 10N HCl q.s. ad pH 2.5 to form a yellow color solution. The solution contained approximately 72 grams of antacid suspension per 100 ml of solution and approximately 43 grams of aluminium hydroxide per 600 ml of solution. The solution was spray dried to yied a non-flowing white powder.

## EXAMPLE 9

In this example, (12 fl oz (US)) 355 ml of commercial RIOPAN Extra Strength suspension were admixed with 32 grams of amorphous silicon dioxide and then acidified with 10N HCl q.s. ad pH 2.5 to yield an off white dispersion. The white dispersion contained approximately 78 grams of antacid suspension per 100 ml. of white dispersion and approximately 13 grams of magaldrate per 100 ml of white dispersion. The dispersion was spray dried to yield a sticky white semisolid.

## EXAMPLE 10

A placebo without magaldrate and aluminium hydroxide was prepared having the approximate formula of Example 1. In this example, 500 ml of the placebo admixture was acidified with 10N HCl q.s. ad pH 2.5. The admixture was processed through the spray drier but a powder was not obtained.

## EXAMPLE 11

In this example, 600 grams of magaldrate gel which had been spray dried for commercial tablet production, were acidified with 10N HCl q.s. ad pH 2.5 to yield a yellow color solution which spray dried to a fluffy white powder. The yellow solution contained approximately 83 grams of magaldrate per 108 m of yellow solution.

## EXAMPLE 12

In this example, (12 fl oz (US)) 355 ml of commercial MYLANTA suspension acidified with 10N NCl q.s. ad pH 2.5 to yield a translucent dispersion. The translucent dispersion contained approximately 83 grams of antacid suspension per 100 ml of translucent dispersion and approximately 3 grams of aluminium hydroxide per 100 ml. translucent dispersion. The dispersion was spray dried to give an off-white to grey powder.

## EXAMPLE 13

In this example, (12 fl oz (US)) 355 ml of commercial MAALOX suspension acidified with 10N HCl q.s. ad pH 2.5 to yield a near-clear dispersion. The near clear dispersion contained approximately 69 grams of antacid suspension per 100 milliliters of clear dispersion and approximately 3 grams of aluminium-hydroxide per 100 milliliters of near clear dispersion. The dispersion was spray dried to give a fluffy white powder.

EXAMPLE 14

In this example, (12 fl oz (US)) 355 ml of commercial AMPHOJEL suspension acidified with 10N HCl q.s. ad pH 2.5 to yield a translucent dispersion. The translucent dispersion contained approximately 88 grams of antacid suspension per 100 milliliters of translucent dispersion and approximately 5 grams of aluminium hydroxide per 100 milliliters of translucent dispersion. The dispersion was spray dried to give a white powder.

EXAMPLE 15

In this example, 100 grams of a rehydratable magaldrate powder were used. The rehydratable magaldrate powder was prepared in accordance with Example 9 of EP-A-0178895, as follows :

| INGREDIENTS | AMOUNT |
|---|---|
| Magaldrate Gel Sulfate, Potassium Based ( 7.38%) | 90.0 kg |
| Potassium Citrate, NF | 1.47 kg |
| AluminiumHydroxide Gel, Guilini, A671/4 | 1.85 kg |
| Sorbitol Solution, USP | 2.85 kg |

The above listed ingredients were processed as set forth below. Continuous agitation must be maintained throughout the processing.

Step 1. Add the sorbitol solution, USP to a suitable tank equipped with a stirrer.

Step 2. Add the potassium citrate, NF then mix until uniform.

Step 3. Concentrate the magaldrate gel sulfate, potassium base to not less than 24% magaldrate and add to the tank in Step #2 .

Step 4. When approximately half of the concentrated gel has been added to the batch, add the aluminium hydroxide gel to the mixture of Step #3.

Step 5. When all of the gel has been added, obtain an assay of the magaldrate content and adjust the quantities of all ingredients to the theoretical ratios. Mix for 5 minutes after each addition.

Step 6. Spray dry the Step #5 gel mixture at the following conditions :

| | |
|---|---|
| Inlet | 400° C |
| Outlet | 130° C |
| Wheel Speed | 20,000 RPM |

A sample of 100 grams of this rehydratable magaldrate powder was acidified with 10N HCl q.s. ad pH 2.5 to yield an opaque liquid. The opaque liquid contained approximately 95 grams of rehydratable magaldrate powder per 100 milliliters of opaque liquid and 62 grams of magaldrate per 100 milliliters of opaque liquid. The liquid was spray dried to give a white powder.

Examples 8 through 15 are summarized with respect to antacid content in Table 5.

## T A B L E  5

| | Grams Antacid Suspension per 100 ml Acidified Composition | Grams Al(OH)₃ per 100 ml Acidified Composition | Grams Magaldrate per 100 ml Acidified Composition |
|---|---|---|---|
| MAALOX TC | 72 | 43 | |
| RIOPAN Extra Stength | 78 | | 13 |
| Spray Dried Magaldrate Gel | 83 | | 83 |
| MYLANTA | 83 | 3 | |
| MAALOX | 69 | 3 | |
| AMPHOJEL | 88 | 5 | |
| Rehydratable Magaldrate Powder | 95 | | 62 |

Instead of spray drying the acidified liquids as described in Examples 8 through 15, the liquids can be admixed with liquid or dry excipients and subjected to tray, drum, spin or flash drying.

EXAMPLE 16

The spray dried powders of Examples 8 through 15 were tested for cytoprotective activity in the ethanol-induced lesion assay as described in Example 7.

The respective powders were admixed with sufficient water to form a liquid and the animals were dosed 400 milligrams of powder per kilo of body weight per oz. in the test. The results are shown in Table 6 below:

## T A B L E  6

| Example | Antacid | %Inhibition |
|---|---|---|
| 8 | MAALOX TC | 87% |
| 9 | RIOPAN Extra Strength | 89% |
| 10 | Placebo | 0% |
| 11 | Spray Dried Magaldrate Gel | 97% |
| 12 | MYLANTA | 82% |
| 13 | MAALOX | 98% |
| 14 | AMPHOJEL | 98% |
| 15 | Rehydratable Magaldrate | 80% |

EXAMPLE 17

In this example, a 100 gram sample of magaldrate gel which had been spray dried to manufacture commercial magaldrate tablets was acidified q.s. ad pH 2.5 with the various acids as shown in Table 7.

## T A B L E  7

Hydrochloric acid:      Yields a yellow solution

Phosphoric acid:      Yields a clear water white solution

Sulfuric acid:      Yields a clear water white solution with an exothermic reaction.  High concentrations of acid produce a charred mass.

Nitric acid:      Yields a near clear white dispersion.

Acetic acid:      Yields a translucent to opaque dispersion. This dispersion when further treated with hydrochloric acid yields a near clear light yelow color dispersion.

Lactic acid:      Yields an opaque suspension.

Citric acid:      Yields a translucent to opaque suspension.

EXAMPLE 18

In this example, a combination dosage form is prepared containing aspirin® and the spray dried acidified magaldrate gel of Example 11 with the ingredients listed below loaded into a hard gelatin capsule in the indicated amounts.

| Ingredients | Amount |
| --- | --- |
| Aspirin®, USP | 600 mg |
| Product of Example 11 | 100 mg |
| Lactose, USP | 200 mg |

The above ingredients are mixed in a V-Blender and then compressed to fill a No. O hard gelatin capsule.

EXAMPLE 19

In this example, a combination dosage form is prepared containing acetaminaphin and the spray dried acidified magaldrate gel of Example 11 with the ingredients listed beow compressed into a tablet.

13

| Ingredients | Amount |
|---|---|
| Acetaminophen, USP | 325 mg |
| Product of Example 11 | 100 mg |
| Microcrystalline cellulose, NF | 200 mg |
| Stearic acid, NF | 10 mg |

The above ingredients are mixed in a V-Bender and then compressed in a tablet press to form a tablet.

EXAMPLE 20

In this example, a combination dosage form is prepared containing ibuprofen and the spray dried acidified magaldrate gel of Example 11 with the ingredients listed below loaded into a hard gelating capsule in the indicated amounts.

| Ingredients | Amount |
|---|---|
| Ibuprofen | 200 mg |
| Product of Example 11 | 100 mg |
| Starch | 200 mg |

The above ingredients are mixed in a V-Blender and then compressed to fill a No. 4 hard gelatin capsules.

Claims

Claims for the Contracting States : BE CH DE FR GB IT LI LU NL SE

1. A cytoprotective pharmaceutical composition characterised in that the composition comprises an exogenously acidified aluminium base containing composition acidified to a pH between 2.25 to 3.25 at which the aluminium base component is solubilized and having a concentration of 50 to 98 grams of precursor aluminium base containing composition prior to acidification per 100 milliliters of acidified composition.

2. A cytoprotective composition as claimed in Claim 1 characterised in that the exogenously acidified aluminium base has been obtained by exogenous acidification of a precursor aluminium base containing antacid composition.

3. A cytoprotective composition as claimed in Claim 1 or Claim 2 characterised in that the composition has been acidified with hydrochloric acid.

4. A cytoprotective composition as claimed in any one of the preceding claims characterised in that the aluminium base is aluminium hydroxide, aluminium hydroxide gel, magaldrate or magaldrate gel.

5. A cytoprotective composition as claimed in any one of claims 1-3, characterised in that the composition also contains solubilized magnesium hydroxide.

6. A cytoprotective composition as claimed in any one of the preceding claims characterised in that the aluminium base containing composition is a commercial antacid composition.

7. A cytoprotective composition as claimed in any one of the preceding claims characterised in that the composition is a spray dried solid.

8. A cytoprotective composition as claimed in any one of the preceding claims, characterised in that the composition also includes an anti-ulcer drug, an anti-spasmodic, an anti-inflammatory agent or an analgesic agent.

9. A process for the preparation of a cytoprotective pharmaceutical composition characterised in that an aluminium base containing antacid composition containing from 50 to 98 grams of aluminium base component per 100 ml is acidified to a pH of 2.25 to 3.25 at which the aluminium base component is solubilized.

10. A process as claimed in Claim 9, characterised in that a composition as claimed in any one of Claims 1 to 8 is prepared.

11. The use of a composition as defined in any one of claims 1 to 8 for the manufacture of a medicament for inducing cytoprotection in humans by increasing the natural defences of the gastrointestinal mucosa.

## Claims for the Contracting State : AT

1. A process for the preparation of a cytoprotective pharmaceutical composition, characterised in that an aluminium base containing antacid composition containing from 50 to 98 grams of aluminium base per 100 ml is exogenously acidified to a pH between 2.25 and 3.25 at which the aluminium base component is solubilised.

2. A process as claimed in Claim 1 characterised in that the composition is acidified with hydrochloric acid.

3. A process as claimed in Claim 1 or Claim 2, characterised in that the aluminium base is aluminium hydroxide, aluminium hydroxide gel, magaldrate or magaldrate gel.

4. A process as claimed in Claim 1 or Claim 2, characterised in that the composition also contains solubilised magnesium hydroxide.

5. A process as claimed in any one of the preceding claims characterised in that the aluminium base containing composition is a commercial antacid composition.

6. A process as claimed in any one of the preceding claims, characterised in that the composition is subsequently dried.

7. A process as claimed in Claim 6 characterised in that after the drying an anti-ulcer agent, an anti-spasmodic, an anti-inflammatary agent or an analgesic agent is incorporated in the composition.

8. The use of a cytoprotective pharmaceutical composition comprising an exogenously acidified aluminium base containing composition acidified to a pH of 2.25 to 3.25 at which the aluminium base component is solubilised and having a concentration of 50 to 98 grams of precursor aluminium base containing composition prior to acidification per 100 ml of acidified composition, for the manufacture of a medicament for inducing cytoprotection in humans by increasing the natural defences of the gastrointestinal mucosa.

## Ansprüche

## Patentansprüche für die Vertragsstaaten : BE, CH, DE, FR, GB, IT, LI, LU, NL und SE :

1. Pharmazeutische Zellschutzzusammensetzung, dadurch gekennzeichnet, daß die Zusammensetzung eine exogen angesäuerte aluminiumbasenhaltige Zusammensetzung umfaßt, die auf einen pH von 2,25 bis 3,25 angesäuert ist, bei dem die Aluminiumbasenkomponente solubilisiert wird, und eine Konzentration von 50 bis 98 g aluminiumbasenhaltiger Vorläuferzusammensetzung vor dem Ansäuern pro 100 ml angesäuerter Zusammensetzung aufweist.

2. Zellschutzzusammensetzung nach Anspruch 1, dadurch gekennzeichnet, daß die exogen angesäuerte Aluminiumbase durch exogene Ansäuerung einer aluminiumbasenhaltigen Vorläufersäureneutralisatorzusammensetzung erhalten wurde.

3. Zellschutzzusammensetzung nach Anspruch 1 oder 2, dadurch gekennzeichnet, daß die Zusammensetzung mit Salzsäure angesäuert wurde.

4. Zellschutzzusammensetzung nach einem der vorhergehenden Ansprüche, dadurch gekennzeichnet, daß die Aluminiumbase Aluminiumhydroxid, Aluminiumhydroxidgel, Magaldrat oder Magaldratgel ist.

5. Zellschutzzusammensetzung nach einem der Ansprüche 1 bis 3, dadurch gekennzeichnet, daß die Zusammensetzung auch solubilisiertes Magnesiumhydroxid enthält.

6. Zellschutzzusammensetzung nach einem der vorhergehenden Ansprüche, dadurch gekennzeichnet, daß die aluminiumbasenhaltige Zusammensetzung eine handelsübliche Säureneutralisatorzusammensetzung ist.

7. Zellschutzzusammensetzung nach einem der vorhergehenden Ansprüche, dadurch gekennzeichent, daß die Zusammensetzung ein sprühgetrockneter Feststoff ist.

8. Zellschutzzusammensetzung nach einem der vorhergehenden Ansprüche, dadurch gekennzeichnet, daß die Zusammensetzung auch ein Antiulcusarzneimittel, ein antispasmodisches Mittel, ein entzündungshemmendes Mittel oder ein analgetisches Mittel enthält.

9. Verfahren zur Herstellung einer pharmazeutischen Zellschutzzusammensetzung, dadurch gekennzeichnet, daß eine aluminiumbasenhaltige Säureneutralisatorzusammensetzung, die 50 bis 98 g Aluminiumbasenkomponente pro 100 ml enthält, auf einen pH von 2,25 bis 3,25 angesäuert wird, bei dem die Aluminiumbasenkomponente solubilisiert wird.

10. Verfahren nach Anspruch 9, dadurch gekennzeichnet, daß eine Zusammensetzung nach einem der Ansprüche 1 bis 8 hergestellt wird.

11. Verwendung einer Zusammensetzung, wie in einem der Ansprüche 1 bis 8 definiert, zur Herstellung eines Medikaments zum Bewirken von Zellschutz in Menschen durch Erhöhen der natürlichen Abwehrkräfte der Gastrointestinalschleimhaut.

**Patentansprüche für den Vertrasgsstaat : AT**

1. Verfahren zur Herstellung einer pharmazeutischen Zellschutzzusammensetzung, dadurch gekennzeichnet, daß eine aluminiumbasenhaltige Säureneutralisatorzusammensetzung, die 50 bis 98 g Aluminiumbasenkomponente pro 100 ml enthält, exogen auf einen pH von 2,25 bis 3,25 angesäuert wird, bei dem die Aluminiumbasenkomponente solubilisiert wird.

2. Verfahren nach Anspruch 1, dadurch gekennzeichnet, daß die Zusammensetzung mit Salzsäure angesäuert wird.

3. Verfahren nach Anspruch 1 oder 2, dadurch gekennzeichnet, daß die Aluminiumbase Aluminiumhydroxid, Aluminiumhydroxidgel, Magaldrat oder Magaldratgel ist.

4. Verfahren nach Anspruch 1 oder 2, dadurch gekennzeichnet, daß die Zusammensetzung auch solubilisiertes Magnesiumhydroxid enthält.

5. Verfahren nach einem der vorhergehenden Ansprüche, dadurch gekennzeichnet, daß die aluminiumbasenhaltige Zusammensetzung eine handelsübliche Säureneutralisatorzusammensetzung ist.

6. Verfahren nach einem der vorhergehenden Ansprüche, dadurch gekennzeichnet, daß die Zusammensetzung anschließend getrocknet wird.

7. Verfahren nach Anspruch 6, dadurch gekennzeichnet, daß in die Zusammensetzung nach dem Trocknen ein Antiulcusmittel, ein antispasmodisches Mittel, ein entzündungshemmendes Mittel oder ein analgetisches Mittel eingearbeitet wird.

8. Verwendung einer pharmazeutischen Zellschutzzusammensetzung, die eine exogen angesäuerte aluminiumbasenhaltige Zusammensetzung, auf einen pH von 2,25 bis 3,25 angesäuert, bei dem die Aluminiumbasenkomponente solubilisiert wird, und mit einer Konzentration von 50 bis 98 g aluminiumbasenhaltiger Vorläuferzusammensetzung vor Ansäuerung pro 100 ml angesäuerter Zusammensetzung umfaßt, zur Herstellung eines Medikaments zum Bewirken von Zellschutz in Menschen durch Erhöhen der natürlichen Abwehrkräfte der Gastrointestinalschleimhaut

**Revendications**

**Revendications pour les Etats Contractants : BE, CH, DE, FR, GB, IT, LI, LU, NL, SE**

1. Composition pharmaceutique cytoprotectrice, caractérisée en ce que la composition comprend une composition contenant une base d'aluminium acidifiée de façon exogène, acidifiée jusqu'à un pH entre 2,25 et 3,25 auquel le composant base d'aluminium est solubilisé, et ayant une concentration de 50 à 98 g de composition contenant la base d'aluminium précurseur avant l'acidification pour 100 ml de composion acidifiée.

2. Composition cytoprotectrice suivant la revendication 1, caractérisée en ce que la base d'aluminium acidifiée de façon exogène a été obtenue par acidification exogène d'une composition antiacide contenant une base d'aluminium précurseur.

3. Composition cytoprotectrice suivant la revendication 1 ou 2, caractérisée en ce que la composition a été acidifiée avec de l'acide chlorhydrique.

4. Composition cytoprotectrice suivant l'une quelconque des revendications précédentes, caractérisée en ce que la base d'aluminium est l'hydroxyde d'aluminium, du gel d'hydroxyde d'aluminium, le magaldrate ou du gel de magaldrate.

5. Composition cytoprotectrice suivant l'une quelconque des revendications 1 à 3, caractérisée en ce que la composition contient aussi de l'hydroxyde de magnésium solubilisé.

6. Composition cytoprotectrice suivant l'une quelconque des revendications précédentes, caractérisée en ce que la composition contenant une base d'aluminium est une composition antiacide du commerce.

7. Composition cytoprotectrice suivant l'une quelconque des revendications précédentes, caractérisée en ce que la composition est un solide séché par pulvérisation.

8. Composition cytoprotectrice suivant l'une quelconque des revendications précédentes, caractérisée en ce que la composition comprend aussi un médicament antiulcéreux, un antispasmodique, un anti-inflammatoire ou un analgésique.

9. Procédé de préparation d'une composition pharmaceutique cytoprotectrice, caractérisé en ce qu'une composition antiacide contenant une base d'aluminium qui contient de 50 à 98 g de composant base d'aluminium pour 100 ml est acidifiée jusqu'à un pH de 2,25 à 3,25 auquel le composant base d'aluminium est solubilisé.

10. Procédé suivant la revendication 9, caractérisé en ce qu'une composition suivant l'une quelconque des revendications 1 à 8 est préparée.

11. Utilisation d'une composition telle que définie dans l'une quelconque des revendications 1 à 8 pour la préparation d'un médicament pour induire la cytoprotection chez l'être humain en augmentant les défenses naturelles de la muqueuse gastro-intestinale.

**Revendications pour l'Etat Contractant : AT**

1. Procédé de préparation d'une composition pharmaceutique cytoprotectrice, caractérisé en ce qu'une composition antiacide contenant une base d'aluminium qui contient de 50 à 98 g de base d'aluminium pour 100 ml est acidifiée de façon exogène jusqu'à un pH entre 2,25 et 3,25 auquel le composant base d'aluminium est solubilisé.

2. Procédé suivant la revendication 1, caractérisé en ce que la composition est acidifiée avec de l'acide chlorhydrique.

3. Procédé suivant la revendication 1 ou 2, caractérisé en ce que la base d'aluminium est l'hydroxyde d'aluminium, du gel d'hydroxyde d'aluminium, le magaldrate ou du gel de magaldrate.

4. Procédé suivant la revendication 1 ou 2, caractérisé en ce que la composition contient aussi de l'hydroxyde de magnésium solubilisé.

5. Procédé suivant l'une quelconque des revendications précédentes, caractérisé en ce que la composition contenant une base d'aluminium est une composition antiacide du commerce.

6. Procédé suivant l'une quelconque des revendications précédentes, caractérisé en ce que la composition est ensuite séchée.

7. Procédé suivant la revendication 6, caractérisé en ce qu'après le séchage, un médicament antiulcéreux, un antispasmodique, un anti-inflammatoire ou un analgésique est incorporé à la composition.

8. Utilisation d'une composition pharmaceutique cytoprotectrice, comprenant une composition contenant une base d'aluminium acidifiée de façon exogène, acidifiée jusqu'à un pH entre 2,25 et 3,25 auquel le composant base d'aluminium est solubilisé, et ayant une concentration de 50 à 98 g de composition contenant la base d'aluminium précurseur avant l'acidification pour 100 ml de composition acidifiée, pour la préparation d'un médicament pour conduire la cytoprotection chez l'être humain en augmentant les défenses naturelles de la muqueuse gastro-intestinale.